# EUROPEAN PATENT APPLICATION

(11) **EP 4 712 023 A1**
(43) Date of publication of application: **18.03.2026**
(21) Application number: 24200778.9
(22) Date of filing: 17.09.2024
(51) Int. Cl.: G06T 7/33

(54) **TECHNIQUE FOR IMAGE DATA REGISTRATION**

(71) Applicant: Stryker European Operations Limited, Carrigtwohill, Co. Cork T45 HX08 (IE)
(72) Inventor: HERRMANN, Dr. Florian, 77963 Schwanau (DE)
(74) Representative: Röthinger, Rainer

(57) **Abstract**

A computer-implemented method for image data registration is provided. The method comprises receiving first image data. The first image data are indicative of bone and of at least one bone marker placed therein, the bone marker having a longitudinal axis. The method also comprises determining a first orientation of the longitudinal axis of the at least one bone marker based on the first image data, receiving second image data indicative of the bone and of the at least one bone marker placed therein, and determining a second orientation of the longitudinal axis of the at least one bone marker based on the second image data. Further still, the method comprises registering, based at least one the first and second orientation of the longitudinal axis of the at least one bone marker, a first local coordinate system associated with at least a segment of the first image data within a global coordinate system.

## Description

### Technical Field

The present disclosure generally relates to medical imaging. In particular, a method for image data registration is presented. Further, a computer program product, an apparatus and a system for image data registration are provided.

### Background

Medical imaging devices have a variety of applications in modern medicine. For example, images generated by medical imaging devices may be used for planning a surgery, navigation during a surgery and pre- or intra-operatively analysing a state of a patient.

In the context of navigated or robot-guided surgery, medical imaging devices are often used to generate image data that will need to be registered within a coordinate system associated with, for example, a surgical tracking system. The surgical tracking system can be configured to acquire positional information of one or more of a patient, a surgical instrument or a mobile imaging device in the operating room.

An erroneous or low-quality registration of the image data may result in a surgeon navigating a surgical instrument based on an improper position of a patient anatomy (e.g., a bone) that is visualized based on the image data. In such a case, the surgery may not have the desired result or may even lead to injuries of the patient.

To enable a navigation based on a high-quality visualization, high-resolution image data, e.g., 3-dimensional image data such as Computer Tomography (CT) image data), may be acquired, registered and visualized. Acquiring high-resolution image data is, however, time consuming. Further, for generating high-resolution image data a high radiation dose is required. Still further, since a patient anatomy often moves, or has to be moved, during a surgery, multiple re-registrations are typically necessary over the course of a surgery, wherein for each re-registration new image data has to be acquired. In the case of high-resolution image data, acquiring new image data for repeated re-registrations results in a high radiation exposure of a patient and, thus, an increased the health risk.

To reduce the need of acquiring high-resolution image data multiple times during a surgical intervention, multiple different techniques may be applied. For example, a patient tracker may be rigidly attached to the anatomy of the patient. After an initial registration of the image data, a pose, i.e., a position and an orientation, of the patient tracker may be tracked to update the initial registration and, thus, to reduce or eliminate the need for repeatedly acquiring high-resolution image data. However, in some cases a patient tracker may obstruct the view of a surgeon and may limit access to a surgical site, in particular when multiple patient trackers are used in parallel (e.g., attached to different vertebrae of a patient).

In another example, EP 3 326 564 A1 discloses registering 3-dimensional image data with 2-dimensional image data. Each 2-dimensional image has to capture at least three point-like fiducial markers that are used for registration purposes. However, the fiducial markers may also obstruct the view of a surgeon, similar to a patient tracker. Further, the need of capturing at least three fiducial markers in every 2-dimensional image may require the image to capture an extensive area of a patient, resulting in high radiation exposure of the patient.

Alternatively, a tracked navigation pointer may be used to manually touch predefined points (e.g., of a patient anatomy) so that a prior registration may be verified and, if needed, updated based on positional data generated using the navigation pointer. However, a manually operated navigation pointer increases the cognitive load on the surgeon and is prone to human error. Moreover, it may not be easy to visually locate and then touch the predefined points with the pointer in case of a minimally invasive procedure.

### Summary

There is a need for a technique for an automatic or semi-automatic image data registration that solves one or more of the problems outlined above.

According to a first aspect, a computer-implemented method for image data registration is provided. The method comprises receiving first image data. The first image data are indicative of bone and of at least one bone marker placed therein.

The bone marker has a longitudinal axis. The method further comprises determining a first orientation of the longitudinal axis of the at least one bone marker based on the first image data, receiving second image data indicative of the bone and of the at least one bone marker placed therein, determining a second orientation of the longitudinal axis of the at least one bone marker based on the second image data, and registering, based at least on the first and second orientation of the longitudinal axis of the at least one bone marker, a first local coordinate system associated with at least a segment of the first image data within a further, in particular global coordinate system.

The first (and any further) local coordinate system may be associated with one or more bones or one or more bone parts. The global coordinate system may define a volume that is larger than the first image data segment. The global coordinate system may include a larger portion of the patient than the first image data segment. The global coordinate system may have a spatially fixed relation to one or more of a tracking camera system, a patient tracker and a tracker of a medical imaging device.

The at least one bone marker may be any device placeable in bone and having a longitudinal axis. As an example, the bone marker may comprise a bone screw, a bone peg, or a guide wire (such as a K-wire), or any other implant having a longitudinal axis. The registration based on the first and second orientations of the longitudinal axis of the at least one bone marker may include determining a first pose, i.e., position and orientation, and a second the pose of the at least one bone marker, wherein the first and second poses are determined with 5 degrees of freedom.

In some variants, a patient anatomy may comprise two or more bones that are movable relative to each other, such as the vertebrae. In such variants, the two or more bones may be associated with spatially separate segments of the first image data.

In some variants, at least the first local coordinate system has previously been registered within the global coordinate system in a previous registration, and the step of registering the first local coordinate system with the global coordinate system may comprise updating the previous registration. For example, the registration of the first local coordinate system within the global coordinate system may be a re-registration (e.g., to update the previous registration). Such a re-registration avoids medical staff, e.g., a surgeon, working based on an incorrect registration. The re-registration may be carried out in response to a user's request and/or in predetermined time intervals and/or after a movement of the bone comprising the at least one bone marker has been detected.

Updating the previous registration may comprise registering a second local coordinate system associated with at least a segment of the second image data within the global coordinate system. The segments of the first and second image data may be indicative of the same at least one bone marker. For example, the first and second image data may have been acquired using different medical imaging modalities (e.g., of different resolutions). The first and second image data may be indicative of the same bone with the same at least one bone marker (e.g., bone screw) implemented therein. Registering the second local coordinate system within the global coordinate system enables a comparison between the first orientation and the second orientation of the longitudinal axis of the bone marker within the global coordinate system. Updating the previous registration may thus further comprise updating the registration of the first local coordinate system within the global coordinate system based on a difference between the first orientation and the second orientation of the longitudinal axis within the global coordinate system.

In some variants, there may be no known previous registration of the first local coordinate system within the global coordinate system, or the previous registration may have to be verified. In such variants, the method may further comprise determining the previous registration of the first local coordinate system within the global coordinate system based on data acquired with a tracked navigation pointer. For example, a part of the at least one bone marker, e.g., the head of a bone screw, may be touched with a tip of the tracked navigation pointer. In this scenario, tracking data, e.g., image data indicative of a tracker carried by the navigation pointer and a tracker associated with the global coordinate system (e.g., a patient tracker), may be generated. The previous registration may then be determined based on the generated tracking data and known dimensions of the navigation pointer and, optionally, of the at least one bone marker. In particular, a pose of the at least one bone marker within the global coordinate system may be determined based on the tracking data and the known dimensions. In some variants, alternative image registration techniques known in the art may be used for determining the previous registration.

The global coordinate system may be defined in various ways, such as based on the position of a tracking camera or based on a position of a tracker located within an operating room. For example, the global coordinate system may have an origin that has in a predefined relationship to the tracking camera or the tracker. The global coordinate system may be defined based on a position of a tracker of a medical imaging device. In another variant, the global coordinate system may be defined based on a position of a patient tracker. The patient tracker may be any tracker attachable to a patient (e.g., a tracker that can be attached to bone or to skin).

In some variants, the second image data may be or may comprise 2-dimensional image data. The 2-dimensional image data may comprise at least two images indicative of the bone and of the bone marker. The at least two images may have respectively been taken from at least a first and a second direction relative to the bone, the first direction being different from the second direction. In some variants, the first direction may be perpendicular relative to the second direction, facilitating a determination of the second orientation of the longitudinal axis.

In some variants, the first image data may be or comprise 2-dimensional image data. In some variants, the first image data may comprise 3-dimensional image data. They may have been natively generated as 3-dimensional image data or they may have been reconstructed from multiple 2-dimensional images (e.g., from fluoroscopy image data). The first image data may comprise 3-dimensional CT, image data indicative of the bone and of the bone marker. As such, the first image data may comprise highly detailed (i.e., high-resolution) images allowing high-quality image registrations (e.g., a high-quality previous registration of the first local coordinate system within the global coordinate system). Additionally or alternatively, the first and/or second image data may comprise fluoroscopy image data (e.g., multiple 2-dimensional fluoroscopy images). While the 2-dimensional fluoroscopy image data may not be as detailed as the 3-dimensional CT image data, the acquisition of the fluoroscopy image data may impose less radiation exposure on a patient than the acquisition of the 3-dimensional CT image data. As a result, the previous registration of the first image data, e.g., the 3-dimensional CT image data, may be updated / re-registered based at least in part on newly acquired 2-dimensional fluoroscopy image data and thus impose less radiation exposure on a patient compared to acquiring new 3-dimensional CT image data and updating / re-registering the previous registration based on the newly acquired 3-dimensional CT image data. In another variant, a registration of 2-dimensional high-resolution image data may be updated based on newly acquired low-resolution 2-dimensional image data, wherein the resolution is low compared to the resolution of the previously registered high-resolution image data. As a result, an update (i.e., a re-registration) of the high-resolution image data may impose less radiation exposure on a patient analogously to the preceding example directed at a scenario with 3-dimensional CT image data and 2-dimensional fluoroscopy image data.

In some variants, the method may further comprise determining a first spatial position of at least a part of the at least one bone marker based on the first image data and determining a second spatial position of at least the part of the at least one bone marker based on the second image data. The step of registering the first local coordinate system within the global coordinate system may then be further based at least on the first and second spatial position of the at least part of the at least one bone marker. For example, the bone marker may be a bone screw with a longitudinal axis and the part of the at least one bone marker may be a (e.g., part of) the screw head. The part of the at least one bone marker may be located on-axis or off-axis relative to the longitudinal axis. An off-axis location of the part of the at least one bone marker may allow determining the pose of the at least one bone marker with six degrees of freedom.

In some variants, at least two bone markers may be placed in the bone. Each bone marker may have a respective longitudinal axis. In such variants, the method may comprise determining a respective first and second orientation of the respective longitudinal axis of each of the at least two bone markers based on the first and second image data. Further, the step of registering the first local coordinate system within the global coordinate system may be based at least in part on the respective first and second orientation of the respective longitudinal axis of each of the at least two bone markers. Further, the longitudinal axes of the at least two bone markers may be non-parallel to each other, thus allowing determining the pose of the at least two bone markers within the global coordinate system with six degrees of freedom.

In some variants, the first orientation and the second orientation of the longitudinal axis of the at least one bone marker are determined based on a segmentation of at least one of the first and second image data. The segmentation may be based on an automatic or semi-automatic image segmentation technique. The image segmentation technique, or another image processing technique, may allow an automatic or semi-automatic determination of the longitudinal axis of the at least one bone marker. As a result, an automatic or semi-automatic registration of the first local coordinate system within the global coordinate system becomes possible.

In some variants, the bone indicated in the first and second image data may comprise multiple bone parts that are movable relative to each other. Each of at least two of the multiple bone parts may comprise at least one bone marker placed therein. In such variants, the method may further comprise segmenting the first image data into multiple first image data segments, wherein each first image data segment comprises a respective one of the at least two of the multiple bone parts, and segmenting the second image data into multiple second image data segments, wherein each second image data segment comprises a respective one of the at least two of the multiple bone parts. For example, the first and second image data may comprise multiple vertebrae, wherein at least two of the multiple vertebrae comprise at least one screw placed therein. In this example, each of the image data segments may comprise one vertebra having at least one screw placed therein. In particular, at least two screws are placed within a single vertebra, wherein the at least two screws have been placed so that their longitudinal axes are non-parallel to each other.

In some variants, the method may further comprise associating a dedicated first coordinate system with each of the first image data segments and a dedicated second coordinate system with each of the second image data segments. The method may still further comprise registering at least one of the dedicated first coordinate systems associated with the first image data segments separately from at least another one of the dedicated first coordinate systems associated with the first image data segments. This registration procedure may be based at least on the first and second orientation of the longitudinal axis of the at least one bone marker placed in the respective one of the at least two of the multiple bone parts. Such a respective registration may allow determining (and possibly updating) individual poses for the multiple bone parts that are movable relative to each other.

The method may further comprise visualizing, on a display device, at least the segment of the first image data associated with the registered first local coordinate system. As a result, the 3-dimensional first image data or at least the segment thereof may be displayed to medical staff, e.g., a surgeon, based on a high-quality image registration indicating the current pose of the bone and, optionally, of the at least one bone marker placed therein. Visualizing at least the segment of the first image data associated with the registered first local coordinate system may comprise visualizing the segment based on the previous registration and rearranging the visualized segment based on the updated / re-registered registration.

In some variants, the visualization may comprise re-arranging (e.g., on the display device) one or more of the first image data segments based on the respective registrations of their dedicated one or more first coordinate systems. As a result, a relative movement between multiple bone parts that are movable relative to each other may be displayed, since each visualized first image data segment may be individually re-arranged according to the respective registration(s) of its dedicated first coordinate system.

In some variants, the first image data may be acquired prior to the second image data, and the first local coordinate system may be repeatedly registered based on the repeatedly acquired second image data. As a result, a high-quality registration of the one or more segments of the 3-dimensional first image data may be maintained over the course of a medical procedure, e.g., a surgery, which may require multiple updates (e.g., re-registrations) while the radiation exposure of a patient may be minimized due to using 2-dimensional second image data for updating the registration of the one or more first image data segments.

According to a second aspect a computer program product is provided. The computer program product comprises instructions that, when executed on at least one processor, cause the at least one processor to carry out the method described herein with reference to the first aspect and the various variants thereof.

According to a third aspect, an apparatus for image data registration of first image data is provided. The apparatus comprises a processor configured to receive the first image data. The first image data are indicative of bone and of at least one bone marker placed therein. The bone marker has a longitudinal axis. The processor is further configured to determine a first orientation of the longitudinal axis of the at least one bone marker based on the first image data, receive second image data indicative of the bone and of the at least one bone marker placed therein, determine a second orientation of the longitudinal axis of the at least one bone marker based on the second image data, and register a first local coordinate system associated with at least a segment of the first image data within a global coordinate system based at least on the first and second orientation of the longitudinal axis of the at least one bone marker.

The apparatus may be further configured to carry out the method described herein with reference to the various variants according to the first aspect.

According to a fourth aspect a system is provided. The system comprises the apparatus described herein and a navigation system configured to generate navigation instructions based on the registered at least one segment of the first image data.

The navigation instructions may allow navigation of, e.g., a surgical instrument relative to the bone indicated in the first image data with a high accuracy. The navigation instructions may be visualized on a display device. For example, the navigation instructions may be overlaid over the visualized one or more first image data segments. In other implementations, the navigation instructions are used to control operation of a surgical robot. In such a case, no visualization of the navigation instructions is needed.

### Brief Description of the Drawings

Further details, advantages and aspects of the present disclosure will become apparent from the following embodiments taken in conjunction with the drawings, wherein:
- Fig. 1: shows a system for image data registration of 3-dimensional image data;
- Fig. 2: shows a flow diagram of a computer-implemented method for image data registration;
- Fig. 3A: shows 3-dimensional image data and a local coordinate system associated with the 3-dimensional image data;
- Fig. 3B: shows a part of the system for image data registration of Fig. 1;
- Fig. 4A: shows 2-dimensional image data and a local coordinate system associated with the 2-dimensional image data;
- Fig. 4B: shows a system for image data registration of 2-dimensional image data;
- Figs. 5A-5C: show different steps of a segmentation of 2-dimensional image data indicating multiple bone parts that are movable relative to each other;
- Fig. 6: schematically shows a step of updating the registration of the local coordinate system associated with the 3-dimensional image data within the global coordinate system;
- Fig. 7A: shows 3-dimensional image data visualized on a display; and
- Fig. 7B: shows the 3-dimensional image data of Fig. 7A re-arranged based on an updated registration.

### Detailed Description

In the following description of exemplary embodiments, the same reference numerals are used to denote the same or similar components.

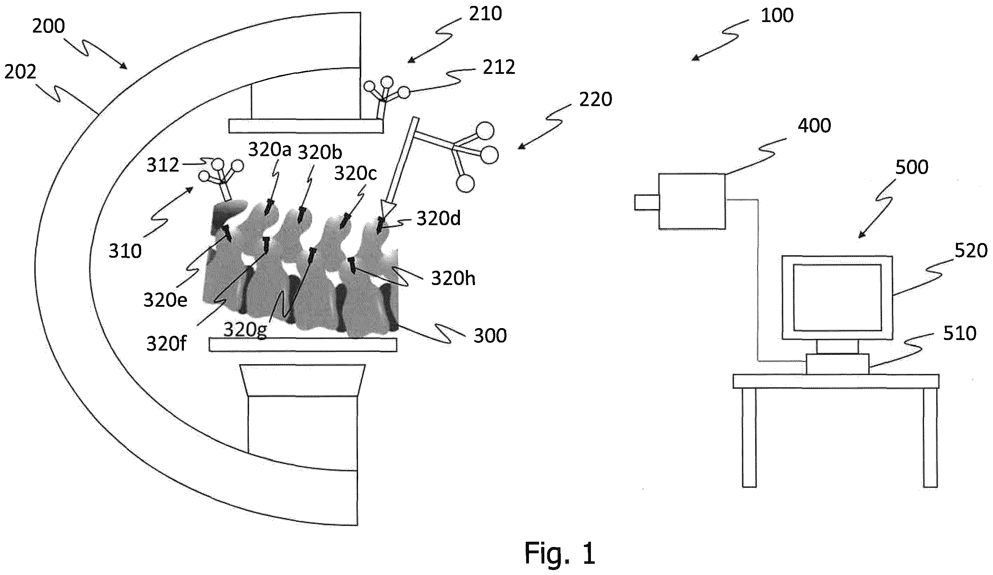

Fig. 1 shows a system 100 for image data registration. The system 100 comprises a medical imaging device 200. The shown medical imaging device 200 comprises a C-arm 202 configured to generate 3-dimensional Computer Tomography (CT) image data. In other variants, the medical imaging device 200 may be configured as a conventional CT scanner. The medical imaging device 200 further comprises a tracker 210 attached thereto. The tracker 210 is an optical tracker comprising at least three tracking markers 212 that are configured to be optically detected by a tracking camera 400 of a tracking system. The tracking camera 400 is configured to generate tracking data, i.e., image data, indicative of the tracking markers 212.

The system 100 further comprises a tracked navigation pointer 220. The tracked navigation pointer 220 may be any commercially available navigation pointer 220. In the shown example, the tracked navigation pointer 220 comprises an optical tracker analogously to the optical tracker 210 of the medical imaging device 200.

Further, Fig. 1 schematically illustrates a patient anatomy in the form of a bone structure 300 with a patient tracker 310 attached thereto. The shown patient tracker 310 is also an optical tracker analogously to the tracker 210 attached to the medical imaging device 200. The bone structure 300 comprises multiple bone markers 320a to 320h placed therein. Each bone marker 320a to 320h is an elongated structure having a longitudinal extension along a longitudinal axis. In the shown example, the bone structure 300 comprises multiple bones (here: vertebrae). Moreover, the bone markers 320a to 320h are screws 320a to 320h each having a screw head and screw shaft defining a longitudinal axis. As can be seen, each of the vertebrae comprises multiple bone screws 320a to 320h.

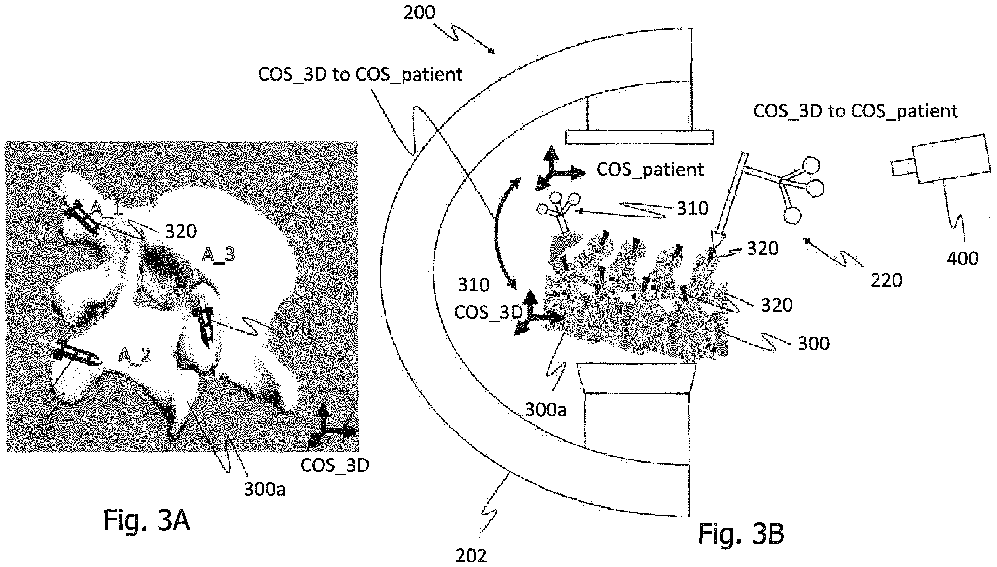

The techniques presented herein will be explained based on examples directed at one or more vertebrae with multiple screws 320a to 320h placed therein as shown in Fig. 1 (see also Figs. 3A to 7B). The corresponding explanations are of an exemplary nature and are provided for an easier understanding, and not for limitation. Other examples are contemplated.

A global coordinate system (not shown) may be defined based on a position of one of the patient tracker 310, the tracker 210 of the medical imaging device 200 and the tracking camera 400. As an example, the global coordinate system may have an origin that has a predefined geometric relationship to one of the patient tracker 310, the tracker 210 of the medical imaging device 200 or the tracking camera 400. In more concrete terms, the origin of the global coordinate system may be rooted in the patient tracker 310 to have a predefined geometric relationship to the respective tracking markers 312.

As further shown in Fig. 1, the navigation pointer 220 may be used to touch the heads of one or more of the screws 320a to 320h while being tracked by the tracking camera 400. In this manner, it becomes possible to determine the positions of the touched screw heads within the global coordinate system (e.g., relative to the patient tracker 310). Since the screws 320a to 320h are made from a radio-opaque material, the screw heads can also be identified in the medical image data acquired by the medical imaging device 200 (e.g., by image processing techniques). By matching the screw head positions as (e.g., automatically) identified in the image data with the screw head positions identified using the navigation pointer 220 in the global coordinate system, the image data acquired by the medical imaging device 200 can initially be registered within the global coordinate system. Of course, there exist other techniques for performing the initial registration.

As becomes apparent from Fig. 1, the system 100 further comprises an apparatus 500 for image data registration and further procedures. Such further procedures comprise tracking procedures and navigation procedures. Of course, these procedures could alternatively be performed by dedicated separate apparatuses.

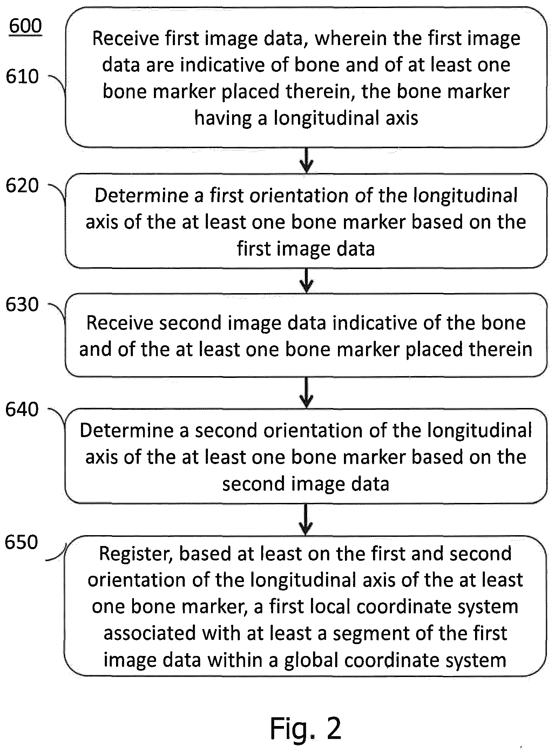

The apparatus 200 of Fig. 1 comprises a processor 510 and a display 520. The processor 510 is configured to carry out various method aspects described herein, e.g., as explained in detail with reference to Figs. 2 to 7B below. The processor 510 is coupled to the tracking camera 400 and configured to process the tracking data generated by the tracking camera 400 to determine the poses of the various trackers 210, 310 in the global coordinate system. The display 520 is coupled to the processor 510 and configured to output visual navigation instructions generated by the processor 510 based, inter alia, on the tracking data.

Fig. 2 shows a flow diagram 600 of a computer-implemented method 600 for image data registration. The method may be executed by the processor 510 of the apparatus 500 shown in Fig. 1 or by any other computing device.

A first step 610 of the method comprises receiving first image data, e.g., directly from the medical imaging device 200 or from a data base in which the image data acquired by the medical imaging device 200 have been stored. According to the example shown in Fig. 1, the received first image data are 3-dimensional CT image data indicative of the bone structure 300 and one or more of the bone markers 320a to 320h placed therein. While some of the examples below are explained with 3-dimensional image data as the first image data, other implementations, e.g., using 2-dimensional image data as the first image data, are contemplated.

An exemplary visualization of 3-dimensional CT image data segment (as acquired, e.g., in a DICOM format) is shown in Fig. 3A. The visualized 3-dimensional CT image data segment is indicative of a single vertebra bone 300a with three screws 320 acting as bone markers being placed therein. Each screw 320 has a longitudinal axis defined by an extension of the respective screw shaft. The 3-dimensional CT image data are associated with, define, or are represented in, a local coordinate system COS_3D.

As schematically shown in Fig. 3B, the local coordinate system COS_3D of the image data of the particular vertebra bone 300a of Fig. 3A may initially be registered within the (exemplary) global coordinate system COS_patient defined by the position of the patient tracker 310. A registration (expressed by the coordinate system transformation "COS_3D to COS_patient" in Fig. 3B) of the local coordinate system COS_3D within the global coordinate system COS_patient may be determined, for example, using the tracked navigation pointer 220 and the camera 400, as explained herein above with reference to Fig. 1. Alternatively, another registration technique may be used, or a previous registration of the first local coordinate system COS_3D within the global coordinate system COS_patient may already be known.

Returning to the flow diagram 600 of Fig. 2, a second method step 620 comprises determining a first orientation of a longitudinal axis of the at least one bone marker 320, 320a to 320h (e.g., a first orientation of the longitudinal axis of one or more of the screws 320, 320a to 320h) based on the 3-dimensional CT image data. The determination of the longitudinal axis may be carried out automatically, e.g., using image processing techniques (such as image segmentation and/or pixel-by-pixel analysis). The corresponding three axes A_1 to A_3 thus determined are exemplarily illustrated in Fig. 3A. It will be appreciated that more or less bone markers 320 may be used, resulting in more or less axes being determined. In case of two or more bone markers 320, the bone markers 320 will in general be placed such that the resulting axes are non-parallel (e.g., oblique) relative to each other.

With reference to Fig. 2, a third method step 630 comprises receiving second image data indicative of the bone structure 300 (i.e., the vertebrae bone 300a) and the at least one bone marker 320, 320a to 320h placed therein. The second image data may be 2-dimensional image data, such as fluoroscopy image data, or a 3-dimensional reconstruction derived from two or more fluoroscopic images taken from different directions. The second (fluoroscopy) image data may have a resolution that is generally lower than the resolution of the first (e.g., CT) image data received in step 610.

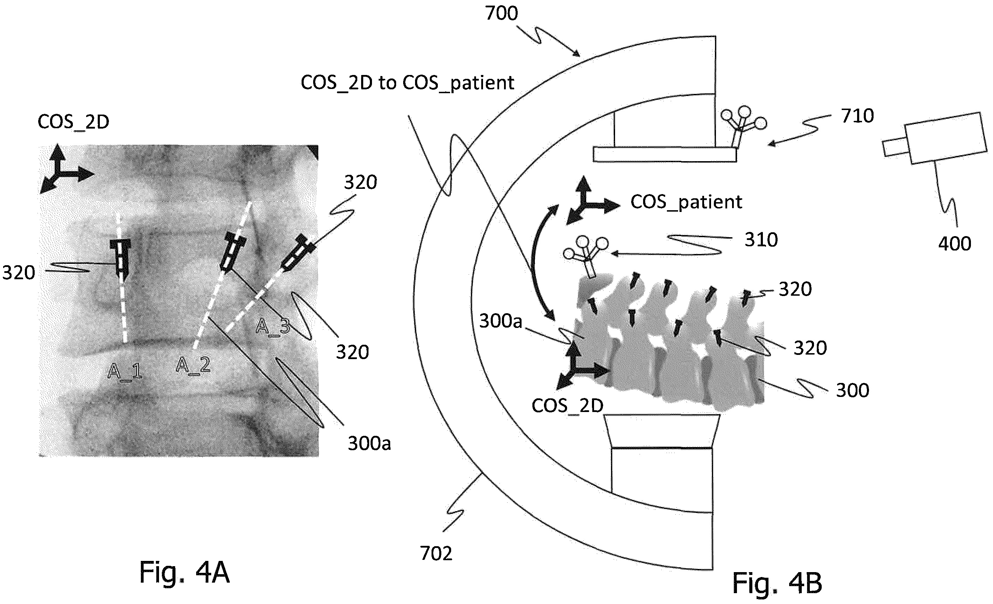

A visual representation of exemplary second image data is shown in Fig. 4A, and Fig.4B shows an exemplary medical imaging device 700 configured to acquire these image data. In the present example, the medical imaging device 700 comprises a C-arm 702 configured to generate 2-dimensional fluoroscopy images.

As becomes apparent from Fig. 4A, the second image data is indicative of the bone structure 300 with multiple vertebrae bones, wherein the central vertebra bone 300a comprises the three bone markers 320, as discussed above. The second image data are associated with (e.g., define or are represented in) a second local coordinate system COS_2D. Here, the term "2D" indicates that while the second local coordinate system can also be a 3-dimensional coordinate system (like the global coordinate system), the second image data represented therein may have been reconstructed from multiple 2-dimensional images. These images have been taken by the imaging device 700 from different directions relative to the bone structure 300 (e.g., from perpendicular directions). The reconstruction may be performed using ray-tracing technique, as generally known in the art. In certain configurations, a single 2-dimensional image may be sufficient for the purposes described herein (e.g., because the bone markers 320 are obliquely arranged relative to each other).

As shown in Fig. 4B, the medical imaging device 700 further comprises a tracker 710 (analogous to the medical imaging device 200 with tracker 210 of Figs 1 and 3B). The tracker 710 is tracked by the tracking camera 400 for registering the second local coordinate system COS_2D within the global coordinate system COS_patient defined by the position of the patient tracker 310. The corresponding registration is defined by a transformation "COS_2D to COS_patient" of the second local coordinate system COS_2D within the global coordinate system COS_patient. The registration may be determined based on image data generated with the camera 400 and indicative of the patient tracker 310 and the tracker 710 of the imaging device 700 for generating the 2-dimensional image data in conjunction with known or previously determined calibration parameters of the imaging device 700 for generating the 2-dimensional image data. The calibration parameters of the imaging device 700 for generating the 2-dimensional image data may be determined based on image data indicative of a calibration phantom, as is generally known in the art.

Registering the local coordinate systems COS_3D and COS_2D within the global coordinate system COS_patient may in each case comprise spatially segmenting (e.g., on a "per bone-basis", such as on a "per vertebra-basis") the 3-dimensional image data (see Fig. 3A) and the 3-dimensionally reconstructed 2-dimensional image data (see Fig. 4A). After segmentation, at least some of the segments, e.g., each segment, may be registered separately.

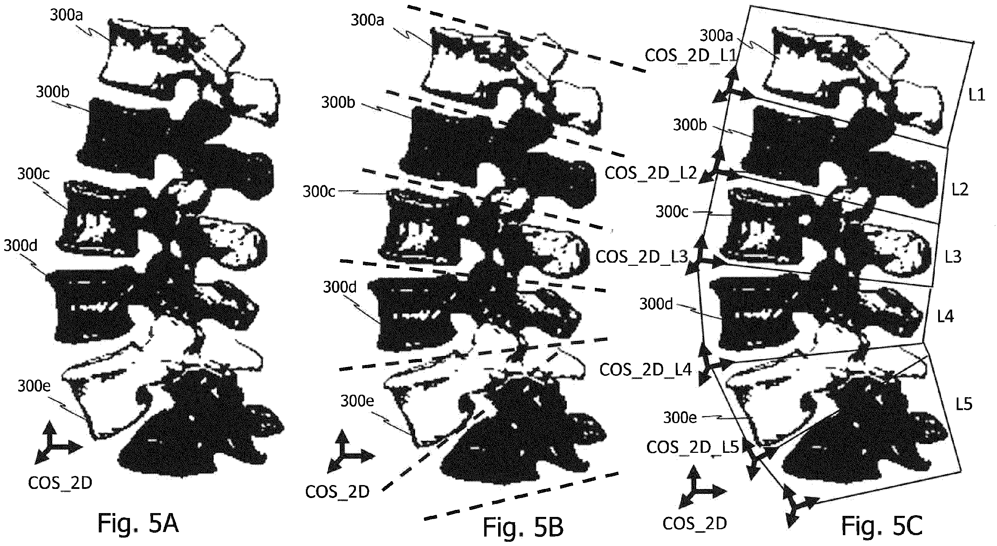

An example of image separation for 3-dimensionally reconstructed 2-dimensional image data is shown in Figs. 5A to 5C. Fig. 5A shows image data indicative of multiple vertebra bones 300a to 300e (e.g., as obtained by 3-dimensionally reconstructed fluoroscopy images generated by the medical imaging device 700). In other implementations not illustrated here, a single 2-dimensional image data set may be sufficient.

As illustrated in Fig. 5B, boundaries between the vertebra bones 300a to 300e are determined first. The boundaries may be determined automatically by image segmentation techniques. As illustrated in Fig. 5C, a 3-dimensional bounding box is then created around each of the vertebra bones 300a to 300e, thus defining an image data segment per vertebra bone 300a to 300e.

For each of the image data segments, a dedicated local coordinate system COS_2D_L1 to COS_2D_L5 may be determined, wherein in the shown example the dedicated local coordinate systems are named based on the imaged vertebra. Each of the dedicated coordinate systems COS_2D_L1 to COS_2D_L5 may then be separately registered within the global coordinate system (e.g., within COS_patient). The same segmentation and registration techniques discussed above with reference to Figs 5A to 5C may be applied to the 3-dimensional first (CT) image data to generate and register dedicated coordinate systems COS_3D_L1 to COS_3D_L5.

Returning to the flow diagram 600 of Fig. 2, a fourth method step 620 comprises determining a second orientation of a longitudinal axis of one or more of the bone markers 320, 320a to 320h based on the second, e.g., 2-dimensional fluoroscopy, image data (in particular based on their 3-dimensional reconstruction). The second orientation may be determined analogously to the first orientation as explained with reference to the second method step 620 of Fig. 2. The corresponding axes A_1 to A_3 representative of the different second orientations are illustrated in Fig. 4A.

A fifth step 650 of the method illustrated in Fig. 2 comprises registering the local coordinate system COS_3D or at least one of the dedicated local coordinate systems COS_3D_L1 to COS_3D_L5 within the global coordinate system (e.g., COS_patient). The registration in step 650 is based at least on the first and second orientation of the longitudinal axis of the at least one bone marker 320, 320a to 320h (i.e., the screws 320, 320a to 320h) as illustrated in Figs. 3A and 4A.

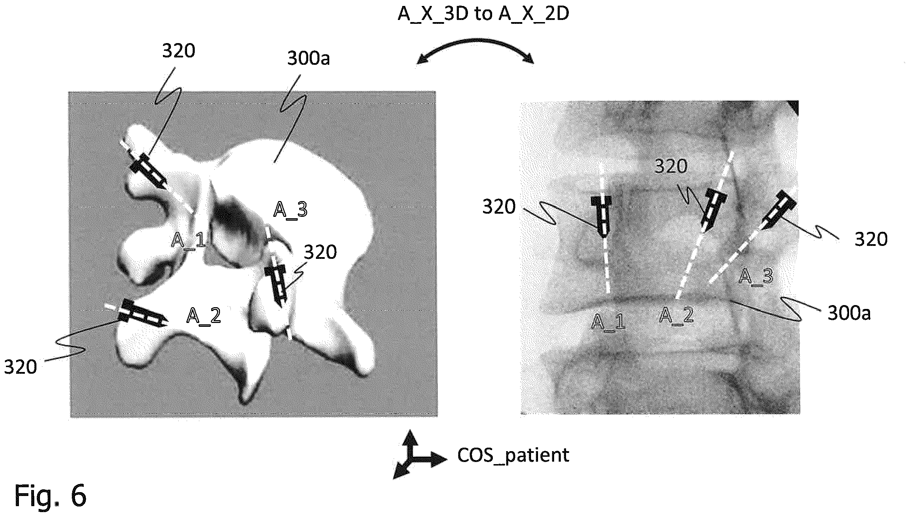

For example, when a previous registration of the first local coordinate system COS_3D exists and the second local coordinate system COS_2D is registered within the global coordinate system, the first and second orientation of the respective longitudinal axis at least one of the bone markers 320, 320a to 320h in the global coordinate system may be compared to each other. The comparison may comprise determining a transformation A_X_3D to A_X_2D that brings the first orientation for a dedicated bone marker 320, 320a to 320h in congruence with the second orientation thereof, as shown in Fig. 6. The transformation may be determined for each of the three bone markers 320 illustrated in Fig. 6 (resulting in the three transformations A_1_3D to A_1_2D, A_2_3D to A_2_2D, A_3_3D to A_3_2D). Each transformation that is different from an identity matrix (mapping one orientation exactly on the other orientation) indicates that a particular vertebra bone 300a was rotated and/or has moved in a translatory manner compared to a previous (including an initial) registration. This also means that this previous registration needs to be updated.

Once determined, the respective transformation A_1_3D to A_1_2D, A_2_3D to A_2_2D, A_3_3D to A_3_2D may be applied to the previous registration of the first local coordinate system COS_3D to determine an updated registration (i.e., a re-registration) for the first local coordinate system COS_3D within the global coordinate system COS_patient. Additionally or alternatively, a dedicated transformation (A_X_L1_3D to A_X_L1_2D;... ; A_X_L5_3D to A_X_L5_2D) may be determined for one or more, e.g., for each, of the dedicated coordinate systems COS_3D_L1 to COS_3D_L5. The previously determined registrations may be registrations in 5 degrees of freedom.

For surgical procedures requiring registrations in 6 degrees of freedom, the transformation A_X_3D to A_X_2D may be determined to bring the axes of at least two non-parallel of the screws 320 in congruence. Additionally or alternatively, the transformation A_X_3D to A_X_2D may be determined to bring a first location of an off-axis point of a dedicated one of the screws 320, 320a to 320h, e.g., an off-axis point on the screw head determined in the 3-dimensional image data, and the first orientation of the longitudinal axis of the dedicated screw 320, 320a to 320h in congruence with a second location of the off-axis point determined in the 2-dimensional image data and the second orientation of the longitudinal axis of the dedicated screw 320, 320a to 320h. Also, one or more on-axis points (e.g., as defined by a screw head center) may be used for this purpose.

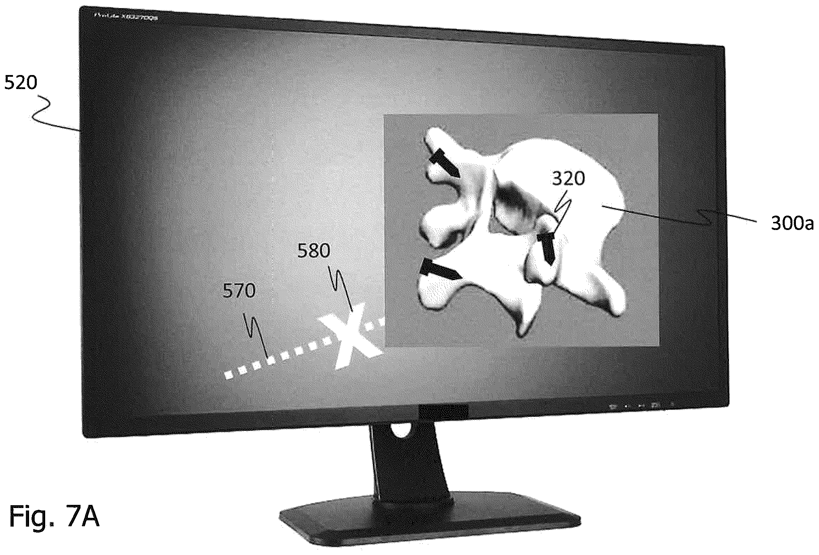

After an initial registration of, for example, the first local coordinate system COS_3D within the global coordinate system COS_patient, the 3-dimensional CT image data may be visualized on the display 520 of Fig. 1 as shown in Fig. 7A. The visualization may further comprise at least one of a visualized trajectory 570 or tip 580 of surgical tool tracked by the tracking camera 500 relative to the vertebra bone 300a. The visualized trajectory 570 or tip 580 serves as a navigation aid.

During a surgical procedure, the vertebra bone 300a may intentionally or unintentionally be moved, which means that the initial registration is no longer valid and the navigation aid as visualized in Fig. 7A potentially inaccurate. In this case, a re-registration is triggered (e.g., by a user or automatically). As a prerequisite of the re-registration, 2-dimensional image data are acquired as discussed above with reference to Figs. 4A and 4B, followed by execution of method step 650 in Fig. 2 to determine an update registration (e.g., as expressed by the transformation A_X_3D to A_X_2D or similar transformations).

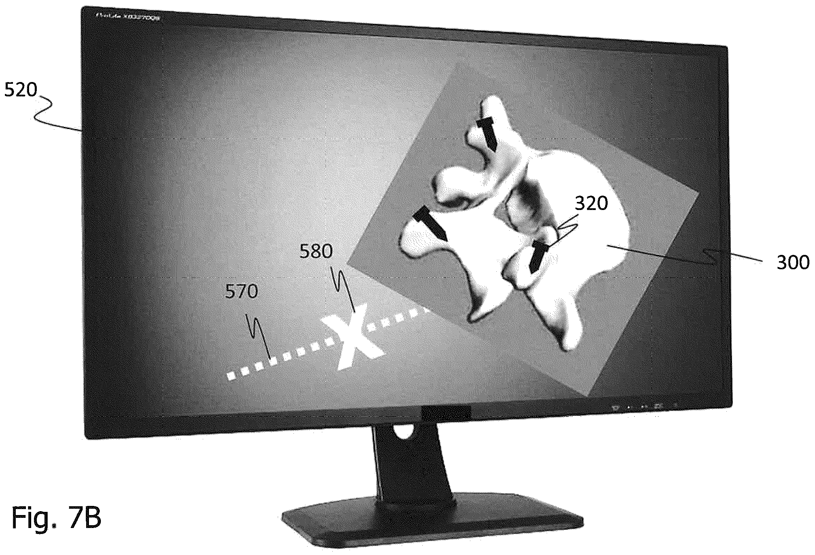

Based on the determined updated registration of the first local coordinate system COS_3D as described with reference to Figs. 2 to 6, the 3-dimensional image data may be visualized, e.g., on the display 520, with an updated orientation as shown in Fig. 7B. In some variants, multiple segments of the 3-dimensional image data may be displayed simultaneously, wherein the orientation of the visualized segments may be updated separately based on the respective registrations determined for the dedicated coordinate systems COS_3D_L1 to COS_3D_L5. In other words, the segments may be moved in reality, and on the display 520, independently from each other.

As has been explained above, embodiments of the technique presented herein enable registrations (in particular updates of previous registrations) of high-quality image data, for example 3-dimensional image data, based on repeatedly generated 2-dimensional image data that impose lower radiation exposure on a patient. Further, a high-quality image with a high-quality registration can be presented to medical staff, e.g., surgeon, at each point of the medical procedure.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A computer-implemented method (600) for image data registration, the method (600) comprising:
receiving (610) first image data, wherein the first image data are data indicative of bone (300) and of at least one bone marker (320, 320a to 320h) placed therein, the bone marker (320, 320a to 320h) having a longitudinal axis;
determining (620) a first orientation of the longitudinal axis of the at least one bone marker (320, 320a to 320h) based on the first image data;
receiving (630) second image data indicative of the bone (300) and of the at least one bone marker (320, 320a to 320h) placed therein;
determining (640) a second orientation of the longitudinal axis of the at least one bone marker (320, 320a to 320h) based on the second image data; and
registering (650), based at least on the first and second orientation of the longitudinal axis of the at least one bone marker (320, 320a to 320h), a first local coordinate system associated with at least a segment of the first image data within a global coordinate system.

2. The computer implemented method (600) according to claim 1, wherein at least the first local coordinate system has previously been registered within the global coordinate system in a previous registration, and wherein the step (650) of registering the first local coordinate system with the global coordinate system comprises updating the previous registration.

3. The computer implemented method (600) according to claim 2, wherein updating the previous registration comprises:
registering a second local coordinate system associated with at least a segment of the second image data within the global coordinate system, wherein the segments of the first and second image data are indicative of the same at least one bone marker (320, 320a to 320h); and
updating the registration of the first local coordinate system within the global coordinate system based on a difference between the first orientation and the second orientation of the longitudinal axis within the global coordinate system.

4. The computer-implemented method (600) according to claim 2 or 3, further comprising:
determining the previous registration of the first local coordinate system within the global coordinate system based on data acquired with a tracked navigation pointer (220).

5. The computer-implemented method (600) according to any preceding claim, wherein
the global coordinate system is defined based on a position of a patient tracker (310).

6. The computer-implemented method (600) according to any preceding claim, wherein
the second image data is 2-dimensional image data and comprises at least two images indicative of the bone (300) and of the bone marker (320, 320a to 320h), wherein the at least two images have respectively been taken from at least a first and a second direction relative to the bone (300), the first direction being different from the second direction.

7. The computer-implemented method (600) according to any preceding claim, wherein
the first image data comprise 3-dimensional Computer Tomography, CT, image data indicative of the bone (300) and of the bone marker (320, 320a to 320h); and/or wherein
the second image data comprise fluoroscopy image data.

8. The computer-implemented method (600) according to any preceding claim, further comprising:
determining a first spatial position of at least a part of the at least one bone marker (320, 320a to 320h) based on the first image data;
determining a second spatial position of at least the part of the at least one bone marker (320, 320a to 320h) based on the second image data; and wherein
the step (650) of registering the first local coordinate system within the global coordinate system is further based at least on the first and second spatial position of the at least part of the at least one bone marker (320, 320a to 320h).

9. The computer-implemented method (600) according to any preceding claim, wherein
at least two bone markers (320, 320a to 320h) are placed in the bone (300), each bone marker (320, 320a to 320h) having a respective longitudinal axis, and wherein the method (600) comprises:
determining a respective first and second orientation of the respective longitudinal axis of each of the at least two bone markers (320, 320a to 320h) based on the first and second image data; and wherein
the step (650) of registering the first local coordinate system within the global coordinate system is based at least in part on the respective first and second orientation of the respective longitudinal axis of each of the at least two bone markers (320, 320a to 320h).

10. The computer-implemented method (600) according to any preceding claim, wherein
the first orientation and the second orientation of the longitudinal axis of the at least one bone marker (320, 320a to 320h) are determined based on a segmentation of the first and second image data.

11. The computer-implemented method (600) according to any preceding claim, wherein
the bone (300) indicated in the first and second image data comprises multiple bone parts (300a to 300e) that are movable relative to each other and wherein each of at least two of the multiple bone parts (300a to 300e) comprises at least one bone marker (320, 320a to 320h) placed therein, the method (600) further comprising:
segmenting the first image data into multiple first image data segments, wherein each first image data segment comprises a respective one of the at least two of the multiple bone parts (300a to 300e); and
segmenting the second image data into multiple second image data segments, wherein each second image data segment comprises a respective one of the at least two of the multiple bone parts (300a to 300e).

12. The computer-implemented method (600) according to claim 11, further comprising:
associating a dedicated first coordinate system with each of the first image data segments and a dedicated second coordinate system with each of the second image data segments.

13. The computer-implemented method (600) according to claim 12, further comprising:
registering at least one of the dedicated first coordinate systems associated with the first image data segments separately from at least another one of the dedicated first coordinate systems associated with the first image data segments based at least on the first and second orientation of the longitudinal axis of the at least one bone marker (320, 320a to 320h) placed in the respective one of the at least two of the multiple bone parts (300a to 300e).

14. The computer-implemented method (600) according to any preceding claim, further comprising:
visualizing, on a display device (520), at least the segment of the first image data associated with the registered first local coordinate system.

15. The computer-implemented method (600) according to claims 13 and 14, wherein
the visualization comprises re-arranging one or more of the first image data segments based on the respective registrations of their dedicated one or more first coordinate systems.

16. The computer-implemented method (600) according to any preceding claim, wherein the first image data is acquired prior to the second image data, and wherein the first local coordinate system is repeatedly registered based on the repeatedly acquired second image data.

17. A computer program product, comprising instructions that, when executed on at least one processor (510), cause the at least one processor (510) to carry out the method (600) of any of claims 1 to 16.

18. An apparatus (500) for image data registration of first image data, the apparatus comprising a processor (520) configured to:
receive (610) first image data, wherein the first image data are indicative of bone and of at least one bone marker (320, 320a to 320h) placed therein, the bone marker (320, 320a to 320h) having a longitudinal axis;
determine (620) a first orientation of the longitudinal axis of the at least one bone marker (320, 320a to 320h) based on the first image data;
receive (630) second image data indicative of the bone and of the at least one bone marker (320, 320a to 320h) placed therein;
determine (640) a second orientation of the longitudinal axis of the at least one bone marker (320, 320a to 320h) based on the second image data; and
register (650) a first local coordinate system associated with at least a segment of the first image data within a global coordinate system based at least on the first and second orientation of the longitudinal axis of the at least one bone marker (320, 320a to 320h).

19. The apparatus (500) according to claim 18, wherein the processor (510) is configured to carry out the method (600) of any of claims 2 to 16.

20. A system (100) comprising:
the apparatus (500) according to claim 18 or 19; and
a navigation system configured to generate navigation instructions based on the registered at least one segment of the first image data.
